# EUROPÄISCHE PATENTSCHRIFT

(11) **EP 0 596 891 B1**
(45) Veröffentlichungstag und Bekanntmachung des Hinweises auf die Patenterteilung: **12.08.1998**
(21) Anmeldenummer: 92910040.2
(22) Anmeldetag: 08.05.1992
(51) Int. Cl.: C07D 285/135, C07D 417/12, A61K 31/41, A61K 31/435

(54) **AMINOALKYLSUBSTITUIERTE 2-AMINO-5-MERCAPTOTHIADIAZOLE, IHRE HERSTELLUNG UND VERWENDUNG**
AMINOALKYL-SUBSTITUTED 2-AMINO-5-MERCAPTOTHIADIAZOLES, THEIR PREPARATION AND THEIR USE
2-AMINO-5-MERCAPTOTHIADIAZOLES A SUBSTITUANT AMINOALKYLE, LEUR FABRICATION ET LEUR UTILISATION

(30) Priorität: 15.06.1991 DE 4119758
(43) Veröffentlichungstag der Anmeldung: 18.05.1994
(73) Patentinhaber: BASF Aktiengesellschaft, 67063 Ludwigshafen (DE)
(72) Erfinder: RENDENBACH-MUELLER, Beatrice, D-6701 Waldsee (DE); TESCHENDORF, Hans-Jürgen, D-6724 Dudenhofen (DE); UNGER, Liliane, D-6700 Ludwigshafen (DE)
(86) Internationale Anmeldenummer: EP9201003
(87) Internationale Veröffentlichungsnummer: WO9222542

(56) Entgegenhaltungen:
- JP-A-62 153 276
- US-A- 4 074 049
- CHEMICAL ABSTRACTS, vol. 107, no. 25, 21. December 1987, Columbus, Ohio, US; abstract no. 136714Q, Seite 791; & JP,A,62 153 276 (YAMANOUCHI PHARMACEUTICAL C O.,LTD.) 08 July 1987

## Beschreibung

Die Erfindung betrifft aminoalkylsubstituierte 2-Amino-5-mercaptothiadiazole, deren Herstellung sowie deren Verwendung zur Bekämpfung von Krankheiten.

In US 4 074 049 werden Aminoalkylthiothiadiazole beschrieben, die fungizide und blutplättchenaggregationshemmende Wirkungen aufweisen. JP 2 153 276 beschreibt ähnliche Verbindungen, die zur Behandlung von Erkrankungen der Leber Verwendung finden.

Es wurde nun gefunden, daß aminoalkylsubstituierte 2-Amino-5-mercapto-1,3,4-thiadiazol-Derivate der Formel I 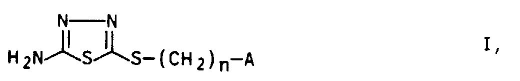 in der
- n: eine ganze Zahl von 2 bis 6 bedeutet und
- A: eine der Gruppen 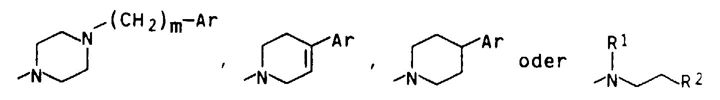 ist, wobei Ar für einen gegebenenfalls einfach durch C₁-C₅-Alkyl, C₁-C₅-Alkoxy, Halogen, Nitro, Hydroxy, Trifluormethyl oder Cyano substituierten Phenylring, einen Pyridyl-, Pyrimidinyl- oder Thienylrest steht, R¹ Wasserstoff oder einen C₁-C₅-Alkylrest darstellt und R² Phenyl, das gegebenenfalls einfach durch C₁-C₅-Alkyl, C₁-C₅-Alkoxy, Halogen, Hydroxy oder Trifluormethyl substituiert ist, oder einen Thienylrest darstellt und m die Zahl 0, 1 oder 2 bedeutet,
sowie deren Salze mit physiologisch verträglichen Säuren interessante pharmakologische Eigenschaften besitzen.

In der Formel I bedeutet A vorzugsweise 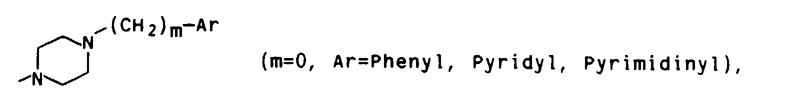 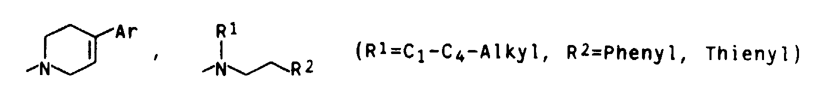 und n vorzugsweise die Zahl 2, 3.

Die Verbindungen der allgemeinen Formel I lassen sich herstellen, indem man
a) Alkylamine der Formel II

   X-(CH₂)ₙ-A II,

   in der n und A wie eingangs definiert sind und X für eine Abgangsgruppe wie Chlor, Brom oder R³SO₂O- steht (mit R³ in der Bedeutung von Niederalkyl oder gegebenenfalls durch C₁-C₃-Alkyl oder Halogen substituiertes Phenyl), oder ein halogenwasserstoffsaures Salz dieser Verbindungen mit 2-Amino-5-mercapto-thiadiazol oder einem Alkalisalz dieser Verbindung umsetzt oder
b) ein Thiadiazol der Formel III 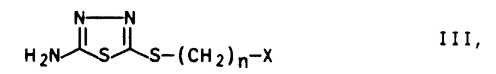 in der n die oben angegebene Bedeutung hat und X wie unter a) definiert ist, mit einem Amin der Formel IV

   H-A IV,

   in der A die angegebenen Bedeutungen hat, umsetzt
und die so erhaltenen Verbindungen gegebenenfalls in ihre Salze mit physiologisch verträgliche Säuren überführt. Im Falle des Verfahrens a) erfolgen die Umsetzungen vorzugsweise in einem Lösungsmittel bei Temperaturen zwischen Raumtemperatur und der Siedetemperatur des verwendeten Lösungsmittels, gegebenenfalls in Gegenwart eines säurebindenden Mittels. Als Lösungsmittel können beispielsweise aliphatische Alkohole, Dimethylformamid, Dimethoxyethan, Tetrahydrofuran, Toluol, Xylol oder ein Keton wie Aceton oder Butanon verwendet werden, als säurebindendes Mittel kommen anorganische Basen wie Natrium- oder Kaliumcarbonat oder organische Basen wie Triethylamin oder Pyridin in Betracht. Letztere können gleichzeitig als Lösungsmittel dienen. Die Isolierung des Rohprodukts erfolgt in üblicher Weise, z.B. durch Filtration, Abdestillieren des Lösungmittels oder Extraktion aus dem Reaktionsgemisch. Eine Reinigung der erhaltenen Verbindung erfolgt in üblicher Weise, beispielsweise durch Umkristallisieren aus einem Lösungsmittel, Chromatographie oder überführen in eine Säureadditionsverbindung.

Die als Ausgangsstoffe verwendeten Alkylamine der Formel II sind literaturbekannt oder können in an sich bekannter Weise durch Alkylierung der Amine der Formel IV mit ω-X-substituierten C₂-C₆-Alkylhalogeniden erhalten werden.

Im Falle des Verfahrens b) erfolgen die Umsetzungen in der Schmelze, gewünschtenfalls auch in Gegenwart eines Lösungsmittels, z.B. Ethylacetat, Tetrahydrofuran, Dimethylformamid, Dimethoxyethan, Toluol oder Xylol bei Temperaturen zwischen Raumtemperatur und dem Siedepunkt des verwendeten Lösungsmittels, vorzugsweise in Gegenwart einer Base wie Natriummethylat, Natriumethylat, Natriumhydrid, Natriumcarbonat, Kaliumcarbonat, eines Amins, z.B. Pyridin. Gegebenenfalls kann auch die Aminkomponente IV im überschuß als Reagenz, Base und Lösungsmittel fungieren.

Gegebenenfalls werden die erhaltenen erfindungsgemäßen Verbindungen in ihre Säureadditionsalze mit physiologisch verträglichen Säuren überführt. Als übliche physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere können aus Fortschritte der Arzneimittelforschung Bd. 10, S. 224 f., Birkhäuser Verlag, Basel und Stuttgart, 1966, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol oder Propanol, einem halogenierten Kohlenwasserstoff wie Methylenchlorid, einem Ether wie Methyl-t-butylether oder Diisopropylether, einem Keton wie Aceton oder Butanon oder einem Ester wie Essigsäureethylester erhalten. Zur besseren Kristallabscheidung können auch Mischungen der genannten Lösungsmittel verwendet werden. Darüberhinaus können pharmazeutisch vertretbare wäßrige Lösungen von Säureadditonsverbindungen der erfindungsgemäßen Verbindungen I durch Auflösen der freien Basen in einer wäßrigen Säurelösung hergestellt werden.

Die erfindungsgemäßen Verbindungen eignen sich zur Bekämpfung von Krankheiten, insbesondere zur Behandlung von Erkrankungen des zentralen Nervensystems (z.B. Parkinsonismus, Schizophrenie) sowie von erhöhtem Blutdruck. Sie weisen insbesondere wertvolle Eigenschaften als Dopaminagonisten, teilweise mit Selektivität für präsynaptische Dopaminrezeptoren, oder als Dopaminantagonisten auf. Die Verbindungen der Formel I zeigen Affinität zum Dopamin-Rezeptor in Rezeptorbindungstests; sie inhibieren die Motilität an Mäusen (gemessen in Lichtschrankenkäfigen) und beeinflussen das Rotationsverhalten an Ratten mit einseitigen 6-Hydroxydopamin-Läsionen der Substantia nigra (Brain Research 24, 485-493, 1970).

Die Wirkungen der neuen Verbindungen lassen sich in Rezeptorbindungstests zeigen:

Striata von Ratten (Sprague Dawley, Charles River) wurden sofort nach Entnahme in 0,32 M Saccharoselösung (0°C) homogenisiert. Das Homogenat wurde filtriert über Gaze, das Filtrat bei 1000 g zentrifugiert (5 min bei 4°C) und der resultierende Überstand bei 40000 g zentrifugiert (10 min, 4°C). Der Rückstand (Membranen) wurde in Inkubationspuffer (50 mM Tris-HCl, 1 mM MgCl₂ und 0,1 % Ascorbinsäure, pH 7,4) aufgenommen und 20 min bei 37°C inkubiert. Anschließend wurde durch Resuspension und Rezentrifugation 2x mit Inkubationspuffer gewaschen. Die Membranen wurden portionsweise in flüssigem Stickstoff eingefroren.

Die Testansätze (1 ml) setzten sich zusammen aus Membranen (380 µg Protein), 1 nM 3H-ADTN (NEN, Dreieich Germany, spez. Radioaktivität 1,4 TBq/mmol) und 0,1 µM SCH 23390 (totale Bindung) oder a) mit zusätzlich 50 nM Spiperon (unspezifische Bindung) oder b) mit Prüfsubstanz. Die Ansätze wurden als Triplikate hergestellt.

Nach beendeter Inkubation (40 min bei 25°C) wurden die Ansätze über Glasfaserfilter (Whatman GF/B) filtriert und kurz mit eiskaltem Waschpuffer (Tris-HCl, pH 7,4) gewaschen. Die auf den Filtern zurückgehaltene Radioaktivität wurde mittels Flüssigkeitszintillationsmessung bestimmt. Die unspezifische Bindung betrug ca. 40 - 50 % der totalen Bindung.

Die Auswertung der Kompetitionskurven und die Bestimmung der Dissoziationskonstante erfolgte über iterative nichtlineare Regressionsanalyse in Anlehnung an das Programm "Ligand" (Muson und Rodbard: Anal. Biochem. 107, 220, 1980).

Affinität der Prüfsubstanzen zum Dopamin-D₂-Rezeptor

| Beispiel | Ki (nM) |
|---|---|
| 1 | 125 |
| 3 | 12 |
| 4 | 6 |
| 8 | 45 |
| 9 | 45 |
| 11 | 15 |
| 14 | 36 |

Die erfindungsgemäßen Verbindungen können in üblicher Weise oral oder parenteral (subkutan, intravenös, intramuskulär, intraperitoneal) verabfolgt werden. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche Wirkstoffdosis etwa 10 bis 500 mg pro Patient und Tag bei oraler Gabe und etwa 1 bis 50 mg pro Patient und Tag bei parenteraler Gabe.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidatien und/oder Treibgasen verarbeitet werden (vgl. H. Sucker et al: Pharmazeutische Technologie, Thieme-Verlag, Stuttgart, 1978). Die so erhaltenen Applikationen enthalten den Wirkstoff normalerweise in einer Menge von 1 bis 99 Gew.-%.

Die folgenden Beispiele sollen die Erfindung näher erläutern:

### Beispiel 1

### 2-Amino-5-[3-(4-fluorphenylpiperazinyl)propylthio]-1,3,4-thiadiazol-dihydrochlorid

4,0 g 2-Amino-5-mercapto-1,3,4-thiadiazol und 11 g Kaliumcarbonat wurden in 50 ml Dimethylformamid vorgelegt und nach Zugabe von 7,6 g 1-(3-Chlorpropyl)-4-(4-fluorphenyl)-piperazin 30 min auf 100°C erhitzt. Die Mischung wurde nach dem Abkühlen auf Eiswasser gegossen. Der ausgefallene Feststoff wurde abgesaugt und durch Kristallisation aus Essigsäureethylester und Ethanol gereinigt. Das Kristallisat wurde in Methanol gelöst, mit etherischer HCI versetzt und das Dihydrochlorid durch Zusatz von Methyl-t-butylether ausgefällt. Der Feststoff wurde abgesaugt und im Vakuum getrocknet.
Ausbeute 3,8 g (30 %) Fp.: 209 - 211°C

### Beispiel 2

### 2-Amino-5-[2-(2-phenylethyl-n-propylamino)-ethylthio]-1,3,4-thiadiazol-dihydrochlorid

4 g 2-Amino-5-mercapto-1,3,4-thiadiazol, 12,5 g Kaliumcarbonat und 7,0 g 2-Chlorethyl-n-propyl-phenethylamin wurden in 10 ml Dimethylformamid 30 min bei 100°C gerührt. Nach dem Abkühlen wurde auf Eiswasser gegossen, der ausgefallene braune Feststoff abgesaugt und in Methyl-t-butylether/ Wasser verteilt. Die organische Phase wurde abgetrennt, mit Wasser gewaschen und eingeengt. Der Rückstand wurde in 2N HCI aufgenommen und mit Methylenchlorid extrahiert. Die wäßrige Phase wurde mit 2N NaOH auf pH 7 gestellt, der ausfallende weiße Feststoff wurde abgesaugt, in i-Propanol suspendiert und mit etherischer HCI versetzt. Der durch Zugabe von Methyl-t-butylether ausgefällte weiße Feststoff wurde abgesaugt und im Vakuum getrocknet.
Ausbeute: 5,7 g (47%) Fp.: 100 - 104°C

### Analog Beispiel 1 bzw. 2 wurden hergestellt:

3. 2-Amino-5-[2-(4-phenyl-1,2,3,6-tetrahydropyridyl)-ethylthio]-1,3,4-thiaadiazol-dihydrochlorid
   Ausbeute: 29 % Fp.: 205 - 206°C
4. 2-Amino-5-[3-(4-phenyl-1,2,3,6-tetrahydropyridyl)-propylthio]-1,3,4-thiadiazol-hydrochlorid
   Ausbeute: 45 % Fp.: 192°C
5. 2-Amino-5-[2-(4-phenylpiperazinyl)-ethylthio]-1,3,4-thiadiazol-hydrochlorid
   Ausbeute: 27 % Fp.: 123 - 124°C
6. 2-Amino-5-[2-(4-pyrid-2-ylpiperazinyl)-ethylthio]-1,3,4thiadiazol-dihydrochlorid
   Ausbeute: 46 % Fp.: 164°C
7. 2-Amino-5-[2-(4-pyrimidin-2-ylpiperazinyl)-ethylthio]-1,3,4-thiadiazol -dihydrochlorid
   Ausbeute: 69 % Fp.: 208°C
8. 2-Amino-5-[2-(4-phenylpiperidinyl)-ethylthio]-1,3,4-thiadiazol-hydrochlorid
   Ausbeute: 62 % Fp.: 180 - 181°C
9. 2-Amino-5-[3-(2-phenylethyl-n-propylamino)-propylthio]-1,3,4-thiadiazol-tartrat
   Ausbeute: 32 % Fp.: 145 - 146°C
10. 2-Amino-5-[2-(4-(2-nitrophenyl)-piperazinyl)-ethylthio]-1,3,4-thiadiazol-hydrochlorid
   Ausbeute: 36 % Fp.: 210 - 211°C
11. 2-Amino-5-[2-(4-(2-ethoxyphenyl)-piperazinyl)-ethylthio]-1,3,4-thiadiazol-hydrochlorid
   Ausbeute: 54 % Fp.: 210°C
12. 2-Amino-5-[2-(4-(4-chlorphenyl)-piperazinyl)-ethylthio]-1,3,4-thiadiazol-hydrochlorid
   Ausbeute: 60 % Fp.: 212 - 213°C
13. 2-Amino-5-[3-(4-(3-methoxyphenyl)-piperazinyl)-propylthio]-1,3,4-thiadiazol-hydrochlorid
   Ausbeute: 58 % Fp.: 212°C
14. 2-Amino-5-[3-(4-(3-methylphenyl)-piperazinyl)-propylthio]-1,3,4-thiadiazol-hydrochlorid
   Ausbeute: 54 % Fp.: 225 - 226°C
15. 2-Amino-5-[3-(4-(3-chlorphenyl)-piperazinyl)-propylthio]-1,3,4-thiadiazol-hydrochlorid
   Ausbeute: 55 % Fp.: 187°C
16. 2-Amino-5-[3-(4-phenethylpiperazinyl)-propylthio]-1,3,4-thiadiazol-hydrochlorid
   Ausbeute: 50 % Fp.: 194 - 195°C
17. 2-Amino-5-[2-(4-(2-methoxyphenyl)-piperazinyl)-ethylthio]-1,3,4-thiadiazol-hydrochlorid
   Ausbeute: 52 % Fp.: 182 - 183°C
18. 2-Amino-5-[2-(4-(2-cyanophenyl)-piperazinyl)-ethylthio]-1,3,4-thiadiazol-hydrochlorid
   Ausbeute: 37 % Fp.: 211 - 212°C
19. 2-Amino-5-[2-(4-(3-trifluormethylphenyl)-piperazinyl)-ethylthio]-1,3,4-thiadiazol-hydrochlorid
   Ausbeute: 28 % Fp.: 145 - 146°C

A) Beispiele für galenische Applikationsformen:
Auf einer Tablettenpresse werden in üblicher Weise Tabletten folgender Zusammensetzung gepreßt.

| | |
|---|---|
| 40 mg | Substanz des Beispiels 1 |
| 120 mg | Maisstärke |
| 13,5 mg | Gelatine |
| 45 mg | Milchzucker |
| 2,25 mg | Aerosil+ (chemisch reine Kieselsäure in submikroskopisch feiner Verteilung) |
| 6,75 mg | Kartoffelstärke (als 6 %iger Kleister) |

B)

| | |
|---|---|
| 20 mg | Substanz des Beispiels 4 |
| 60 mg | Kernmasse |
| 60 mg | Verzuckerungsmasse |

Die Kernmasse besteht aus 9 Teilen Maisstärke, 3 Teilen Milchzucker und 1 Teil Vinylpyrrolidon-vinylacetat-Mischpolymerisat 60:40. Die Verzuckerungsmasse besteht aus 5 Teilen Rohrzucker, 2 Teilen Maisstärke, 2 Teilen Calciumcarbonat und 1 Teil Kalk. Die so hergestellten Dragees werden anschließend mit einem magensaftresistenten überzug versehen.
C) 10 g Substanz des Beispiels 2 werden in 5000 ml Wasser unter Zusatz von NaCl gelöst und mit 0,1N NaOH auf pH 6,0 eingestellt, so daß eine blutisotonische Lösung entsteht. Jeweils 1 ml dieser Lösung wird in Ampullen gefüllt und sterilisiert.

## Patentansprüche

1. Aminoalkylsubstituierte 2-Amino-5-mercapto-1,3,4-thiadiazol-Derivate der Formel I 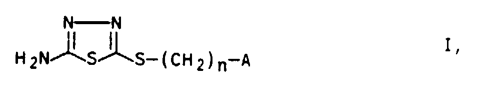 in der
n eine ganze Zahl von 2 bis 6 bedeutet und
A eine der Gruppen 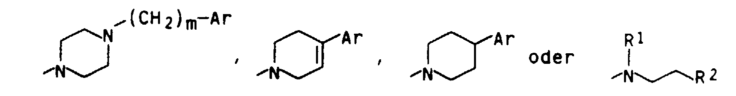 ist, wobei Ar für einen gegebenenfalls einfach durch C₁-C₅-Alkyl, C₁-C₅-Alkoxy, Halogen, Nitro, Hydroxy, Trifluormethyl oder Cyano substituierten Phenylring, einen Pyridyl-, Pyrimidinyl- oder Thienylrest steht, R¹ Wasserstoff oder einen C₁-C₅-Alkylrest darstellt und R² Phenyl, das gegebenenfalls einfach durch C₁-C₅-Alkyl, C₁-C₅-Alkoxy, Halogen, Hydroxy oder Trifluormethyl substituiert ist, oder einen Thienylrest darstellt und m die Zahl 0, 1 oder 2 bedeutet,
sowie deren Salze mit physiologisch verträglichen Säuren.

2. Verfahren zur Herstellung der aminoalkylsubstituierten 2-Amino-5-mercapto-1,3,4-thiadiazol-Derivate der Formel I gemäß Anspruch 1, dadurch gekennzeichnet, daß man
a) Alkylamine der Formel II
X-(CH₂)ₙ-A II,
in der n und A wie eingangs definiert sind und X für eine Abgangsgruppe wie Chlor, Brom oder R³SO₂O- steht (mit R³ in der Bedeutung von Niederalkyl oder ggf. durch C1-3-Alkyl oder Halogen substituiertes Phenyl) oder ein halogenwasserstoffsaures Salz dieser Verbindungen mit 2-Amino-5-mercapto-1,3,4-thiadiazol oder einem Alkalisalz dieser Verbindung umsetzt oder
b) ein Thiadiazol der Formel III 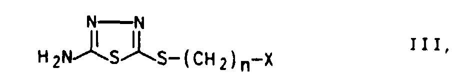 in der n die oben angegebene Bedeutung hat und X wie unter a) definiert ist mit einem Amin der Formel IV,
H-A IV,
in der A die angegebene Bedeutung hat, umsetzt
und die so erhaltenen Verbindungen gegebenenfalls in ihre Salze mit physiologisch verträglichen Säuren überführt.

3. 2-Amino-5-mercapto-1,3,4-thiadiazol-Derivate der Formel I gemäß Anspruch 1 zur Verwendung bei der Bekämpfung von Krankheiten.

4. Verwendung eines 2-Amino-5-mercapto-1,3,4-thiadiazol-Derivates der allgemeinen Formel I nach Anspruch 1 zur Herstellung eines Arzneimittels gegen Erkrankungen des zentralen Nervensystems oder gegen Erkrankungen, die mit erhöhtem Blutdruck einhergehen.

## Claims

1. An aminoalkyl-substituted 2-amino-5-mercapto-1,3,4-thiadiazole derivative of the formula I 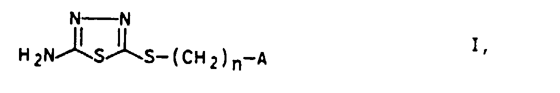 where
n is an integer from 2 to 6, and
A is 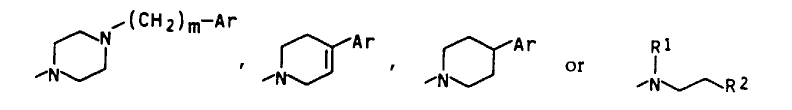 where Ar is phenyl which is unsubstituted or monosubstituted by C₁-C₅-alkyl, C₁-C₅-alkoxy, halogen, nitro, hydroxyl, trifluoromethyl or cyano, or is pyridyl, pyrimidinyl or thienyl, R¹ is hydrogen or C₁-C₅-alkyl, and R² is phenyl which is unsubstituted or monosubstituted by C₁-C₅-alkyl, C₁-C₅-alkoxy, halogen, hydroxyl or trifluoromethyl, or is thienyl, and m is 0, 1 or 2,
and its salts with physiologically tolerated acids.

2. A process for preparing the aminoalkyl-substituted 2-amino-5-mercapto-1,3,4-thiadiazole derivatives of the formula I as claimed in claim 1, which comprises
a) reacting alkylamines of the formula II
X-(CH₂)ₙ-A II,
where n and A are as defined above, and X is a leaving group such as chlorine, bromine or R³SO₂O- (where R³ is lower alkyl or phenyl which is unsubstituted or substituted by C₁-C₃-alkyl or halogen), or a hydrohalic acid salt of these compounds, with 2-amino-5-mercapto-1,3,4-thiadiazole or an alkali metal salt of this compound, or
b) reacting a thiadiazole of the formula III 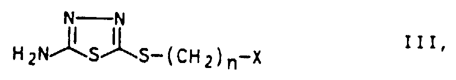 where n has the abovementioned meanings, and X is as defined in a), with an amine of the formula IV
H-A IV,
where A has the stated meanings,
and converting the resulting compounds where appropriate into their salts with physiologically tolerated acids.

3. A 2-amino-5-mercapto-1,3,4-thiadiazole derivative of the formula I as claimed in claim 1 for use for controlling diseases.

4. The use of a 2-amino-5-mercapto-1,3,4-thiadiazole derivative of the formula I as claimed in claim 1 for producing a drug for disorders of the central nervous system or disorders associated with elevated blood pressure.

## Revendications

1. Dérivés du 2-amino-5-mercapto-1,3,4-thiadiazole à substituants aminoalkyle, répondant à la formule I 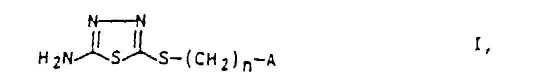 dans laquelle
n est un nombre entier allant de 2 à 6 et
A représente l'un des groupes 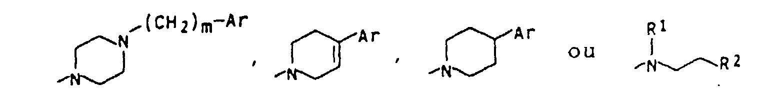 dans lesquels Ar représente un cycle phényle portant éventuellement un substituant alkyle en C1-C4, alcoxy en C1-C5, halogéno, nitro, hydroxy, trifluorométhyle ou cyano, un radical pyridyle, pyrimidinyle ou thiényle,
R¹ représente l'hydrogène ou un groupe alkyle en C1-C5 et
R² représente un groupe phényle, éventuellement substitué une fois par alkyle en C1-C5, alcoxy en C1-C5, halogéno, hydroxy ou trifluorométhyle, ou représente un radical thiényle, et m est égal à 0, 1 ou 2,
et leurs sels d'acides acceptables pour l'usage pharmaceutique.

2. Procédé de préparation des dérivés du 2-amino-5-mercapto-1,3,4-thiadiazole à substituants aminoalkyle de formule I selon la revendication 1, caractérisé par le fait que
a) on fait réagir des alkylamines de formule II
X-(CH₂)ₙ-A II
dans laquelle n et A ont les significations indiquées ci-dessus et X représente un substituant éliminable tel que chloro, bromo ou R³SO₂O- (R³ représentant un groupe alkyle inférieur ou un groupe phényle éventuellement substitué par un groupe alkyle en C1-C3 ou un halogène),
éventuellement à l'état d'halogénhydrates,
avec le 2-amino-5-mercapto-1,3,4-thiadiazole ou l'un de ses sels alcalins,
ou bien
b) on fait réagir un thiadiazole de formule III 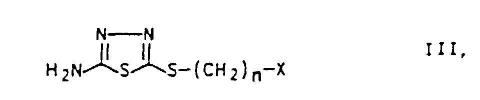 dans laquelle n a les significations indiquées ci-dessus et X a les significations indiquées ci-dessus sous a), avec une amine de formule IV
H-A IV
dans laquelle A a les significations indiquées ci-dessus,
et le cas échéant, on convertit les composés obtenus en leurs sels d'acides acceptables pour l'usage pharmaceutique.

3. Dérivés du 2-amino-5-mercapto-1,3,4-thiadiazole de formule I selon la revendication 1 pour l'utilisation dans le traitement de maladies.

4. Utilisation d'un dérivé du 2-amino-5-mercapto-1,3,4-thiadiazole de formule générale I selon la revendication 1, pour la préparation d'un médicament prévu pour le traitement des maladies du système nerveux central ou des maladies s'accompagnant d'une hypertension.
